# EUROPÄISCHE PATENTSCHRIFT

(11) **EP 1 883 623 B1**
(45) Veröffentlichungstag und Bekanntmachung des Hinweises auf die Patenterteilung: **20.04.2011**
(21) Anmeldenummer: 06754975.8
(22) Anmeldetag: 03.05.2006
(51) Int. Cl.: C07C 253/34, C07C 255/24, C07C 255/25

(54) **VERFAHREN ZUR ISOLIERUNG VON METHYLGLYCINNITRIL-N,N-DIACETONITRILEN AUS EINEM WÄSSRIGEN ROHGEMISCH**
METHOD FOR ISOLATING METHYL GLYCINE NITRILE-N,N-DIACETONITRILES FROM AN AQUEOUS CRUDE MIXTURE
PROCEDE D'ISOLEMENT DE METHYLGLYCINENITRILE-N,N-DIACETONITRILES DANS UN MELANGE BRUT AQUEUX

(30) Priorität: 06.05.2005 DE 102005021056
(43) Veröffentlichungstag der Anmeldung: 06.02.2008
(73) Patentinhaber: BASF SE, 67056 Ludwigshafen (DE)
(72) Erfinder: OFTRING, Alfred, 67098 Bad Dürkheim (DE); JUDAT, Bernd, 68163 Mannheim (DE); RAULS, Matthias, 67061 Ludwigshafen (DE); FRIESE, Katrin, 68163 Mannheim (DE)
(86) Internationale Anmeldenummer: PCT/EP2006/062008
(87) Internationale Veröffentlichungsnummer: WO 2006/120144

(56) Entgegenhaltungen:
- WO-A-01/90041
- DE-A1- 3 246 494
- US-A- 5 849 950
- HILTON A M ET AL: "Emulsion solidification of meta-chloronitrobenzene : Purification and crystallisation" JOURNAL OF CRYSTAL GROWTH, ELSEVIER, AMSTERDAM, NL, Bd. 166, Nr. 1, September 1996 (1996-09), Seiten 971-975, XP004053484 ISSN: 0022-0248

## Beschreibung

Die Erfindung betrifft ein Verfahren zur Isolierung von Methylglycinnitril-N,N-diacetonitril aus einem wässrigen Rohgemisch, wie es bei der Herstellung von Methylglycinnitril-N,N-diacetonitril anfällt.

Die häufig als Komplexbildner in Haushaltsreinigungsmitteln eingesetzten Aminopolyphosphonate, Polycarboxylate oder Aminopolycarboxylate, wie Ethylendiaminotetraessigsäure (EDTA), sind nur in geringem Maße biologisch abbaubar. Eine preiswerte Alternative stellen die Glycin-N,N-Diessigsäurederivate, wie die Methylglycin-N,N-Diessigsäure (MGDA) dar, welche ungiftig und gut biologisch abbaubar ist. Die Verwendung von MGDA und von verwandten Glycin-N,N-Diessigsäurederivaten in Reinigungsmitteln sowie deren Synthesen sind in WO-A 94/29421 und US 5,849,950 beschrieben. Für eine kostengünstige Produktion der Glycin-N,N-Diessigsäurederivate werden hohe Anforderungen an die Ausbeute der einzelnen Syntheseschritte und Reinheit der isolierten Zwischenprodukte gestellt.

MGDA wird durch Umsetzung von Iminodiacetontril mit Acetaldehyd und Blausäure oder von alpha-Alaninnitril mit Formaldehyd und Blausäure und alkalische Hydrolyse des als Zwischenprodukt erhaltenen MGDN mit Natronlauge hergestellt, wobei das Trinatriumsalz des MGDA erhalten wird. Um hohe MGDA-Ausbeuten zu erzielen ist es wünschenswert, MGDN als Zwischenprodukt zu isolieren und als Reinstoff in dem sich anschließenden Hydrolyseschritt einzusetzen. In US 5,849,950, Beispiel 2, wird MGDN aus dem Rohproduktgemisch der Umsetzung von HCN, Formaldehyd und Alaninnitril, welches in einem vorgelagerten Schritt in situ aus Acetaldehyd, HCN und Ammoniak erzeugt wird, durch Kühlung des Produktgemischs auskristallisiert.

Das wässrige, MGDN enthaltende Rohgemisch kann eine Reihe von Nebenkomponenten enthalten. Wird MGDN durch pH-kontrollierte Strecker-Reaktion von Iminodiacetonitril (IDN) mit Acetaldehyd und Blausäure hergestellt, kann IDN entweder als kristalliner Rohstoff eingesetzt oder aber durch pH-kontrollierte Umsetzung von Urotropin mit HCN in wässriger Lösung erzeugt und ohne Isolierung zum MGDN umgesetzt werden. Als Nebenkomponenten enthält das wässrige MGDN-Rohgemisch dann Ammoniumsulfat, Acetaldehydcyanhydrin, Formaldehydcyanhydrin, Methylen-bis-iminodiacetonitril (MBIDN), Nitrilotriacetonitril (NTN), sowie nicht umgesetzte Edukte.

MGDN besitzt eine stark temperaturabhängige Löslichkeit in Wasser. So lassen sich bei 10 °C nur noch etwa 0,5 Gew.-% MGDN in Wasser oder einer wässrigen Ammoniumsulfatlösung lösen. Bei ca. 60 °C beträgt die Löslichkeit noch etwa 5 Gew.-%. Oberhalb von ca. 60 °C liegt eine Emulsion aus MGDN und seiner wässrigen Lösung mit mehr als 5 Gew.% gelöstem MGDN vor. Ein Phasendiagramm des Systems Wasser-MGDN ist nicht bekannt.

Aufgrund der starken Temperaturabhängigkeit der Löslichkeit von MGDN in Wasser kann MGDN durch Kühlungskristallisation und anschließender Fest/Flüssig-Trennung aus seiner wässrigen Lösung isoliert werden. Beim Abkühlen einer wässrigen Lösung oder Emulsion von MGDN fällt MGDN als Feststoff in Form von feinen, nadeligen Kristallen aus, welche agglomerieren. Die in den Agglomeraten eingeschlossene Mutterlauge enthält als Verunreinigungen sämtliche Nebenkomponenten der MGDN-Synthese, so dass das durch Filtration abgetrennte feuchte Kristallisat eine dunkelbraune Farbe aufweist. Bei der Kühlungskristallisation treten zudem Verkrustungen an den Wänden des Kristallisators auf.

Aufgabe der Erfindung ist es, ein verbessertes Verfahren zur Abtrennung von MGDN aus den bei seiner Herstellung anfallenden wässrigen Rohproduktgemischen bereitzustellen.

Gelöst wird die Aufgabe durch ein Verfahren zur Isolierung von Methylglycinnitril-N,N-diacetonitril (MGDN) aus einer MGDN enthaltenden, wässrigen Emulsion mit einem MGDN-Gehalt von 3 - 50 Gew.-% in einem Kristallisator mit den Schritten:
(a) die wässrige Emulsion wird, ausgehend von einer Temperatur oberhalb des Erstarrungspunktes, auf eine Temperatur unterhalb des Erstarrungspunktes abgekühlt, wobei die Abkühlrate im zeitlichen Mittel 5 K/h nicht übersteigt, bis im Wesentlichen die Gesamtmenge des emulgierten MGDN fest geworden ist,
(b) die erhaltene wässrige Suspension wird weiter abgekühlt und/oder aufkonzentriert, wobei die Abkühlrate größer als in Schritt (a) sein kann.

Wesentlich ist, dass in Schritt (a) das MGDN enthaltende Rohproduktgemisch, welches oberhalb des Erstarrungspunktes des MGDN als Emulsion von MGDN als disperse organische Phase in einer gesättigten wässrigen MGDN-Lösung als kontinuierliche wässrige Phase vorliegt, im zeitlichen Mittel nur sehr langsam, also mit einer geringen Abkühlrate (ausgedrückt in K/h) unter den Erstarrungspunkt abgekühlt wird. Der Erstarrungspunkt ist diejenige Temperatur, bei der MGDN von der gegebenen Reinheit fest wird, die in der wässrigen Phase emulgierten MGDN-Tröpfchen also erstarren. Dieser Punkt kann, abhängig von Art und Menge der in dem MGDN enthaltenen Verunreinigungen, um einige °C variieren und liegt für reines MGDN bei ca. 65 °C.

Die Abkühlrate in Schritt (a) ist im zeitlichen Mittel ≤ 5 K/h, vorzugsweise ≤ 3 K/h. Die Abkühlrate kann prinzipiell beliebig klein sein, für praktische Zwecke beträgt sie im Allgemeinen von 1 K/h bis 5 K/h. Die momentane Abkühlrate kann konstant sein oder variieren, sofern bis zu dem Zeitpunkt, zu dem praktisch die Gesamtmenge des emulgierten MGDN vom flüssigen in den festen (kristallinen) Zustand übergegangen ist, sich aus der Emulsion also eine Suspension gebildet hat, die Abkühlrate im zeitlichen Mittel den angegebenen Grenzwert nicht überschreitet. Beispielsweise kann die Emulsion erst schnell abgekühlt werden, mit einer Rate, die den angegebenen Grenzwert momentan deutlich überschreitet, und anschließend mit einer viel geringeren Abkühlrate, die deutlich unterhalb des angegebenen Grenzwertes liegt, weiter abgekühlt werden, oder die Temperatur wird anschließend eine Zeitlang im Wesentlichen konstant gehalten, und zwar bis praktisch das gesamte emulgierte MGDN erstarrt ist. Beispielsweise kann die Emulsion "schnell", z. B. mit einer momentanen Abkühlrate von > 5 K/h, auf eine Temperatur bis zu 10 °C unterhalb des Erstarrungspunktes abgekühlt werden, worauf sich eine "Haltezeit" anschließt, währenddessen die Temperatur im Wesentlichen konstant gehalten wird, bis praktisch das gesamte emulgierte MGDN erstarrt ist. Beispielsweise kann diese Haltezeit von 0,5 bis 2 h betragen.

Die Abkühlung der wässrigen Emulsion in Schritt (a) kann durch Verdampfen lassen von Wasser und/oder durch Abführen von Wärme über die Kristallisatorwand erfolgen.

In dem sich anschließenden Schritt (b) wird die erhaltene wässrige Suspension weiter abgekühlt und/oder aufkonzentriert, wobei die Abkühlrate größer als in Schritt (a) sein kann.

Durch die langsame Abkühlung der Suspension in Schritt (a) wird sichergestellt, dass das emulgierte MGDN bereits erstarrt ist und in kristalliner Form vorliegt, bevor es durch weiteres Abkühlen des MGDN/Wasser-Gemischs in nennenswertem Umfang zur Kristallisation von gelöstem MGDN aus der Lösung kommt. Es werden also die Erstarrung des emulgierten MGDN und die Kristallisation von gelöstem MGDN aus der Lösung zeitlich getrennt. Dadurch wird eine Neubildung von Kristallkeimen weitgehend unterdrückt, wodurch die Bildung von Feinanteil vermieden wird. Die Kristallisation des MGDN aus der Lösung erfolgt dann ganz überwiegend an den bereits schon vorhandenen groben MGDN-Kristallen. Dieser Effekt tritt nicht ein, wenn durch zu schnelles Abkühlen Erstarrung der emulgierten MGDN-Tröpfchen und Kristallisation von gelöstem MGDN im Wesentlichen gleichzeitig erfolgen.

Vorzugsweise wird während des Kristallisationsvorgangs zumindest zeitweise Wasser aus dem wässrigen Gemisch verdampft, wobei dieser Verdampfungsvorgang mit einer Abkühlung und/oder Aufkonzentrierung des Gemischs einhergehen kann. Durch die Verdampfung entsteht, im Wesentlichen angrenzend an die Grenzfläche Flüssigkeit/Gasraum des wässrigen Gemischs, eine Zone der Übersättigung, in der es vermehrt zur Kristallisation von MGDN aus der Lösung kommt. Werden weitere Kristallkeime gebildet, so werden diese nur in der relativ schmalen Zone der Übersättigung in der Nähe der Grenzfläche gebildet, so dass deren Zahl begrenzt wird. Diese werden anschließend in das Innere der Suspension transportiert, wo sie bei nur geringer Übersättigung weiter wachsen.

Abkühlung durch Verdampfen lassen von Wasser ist der Abkühlung durch Abführung von Wärme über die Behälterwand vorzuziehen, solange eine Verdampfungskühlung noch technisch problemlos möglich ist. Dadurch wird auch vermieden, dass sich MGDN-Kristalle an der Kristallisatorwand abscheiden, und es zur Verkrustung der Kristallisatorwand kommt. Problemlos möglich ist eine Verdampfungskühlung im Allgemeinen bis zu einer Temperatur des wässrigen Gemischs von ca. 30 °C.

Es kann das Gemisch abgekühlt werden und der Wasseranteil des Gemischs im Wesentlichen konstant gehalten werden. Abgekühlt wird das Gemisch durch den Entzug von Wärme durch den Verdampfungsvorgang, wenn der Verdampfungsvorgang im Wesentlichen (oder zumindest partiell) adiabatisch durchgeführt wird. Im Wesentlichen konstant gehalten wird der Wasseranteil des MGDN-Gemischs dadurch, dass der Wasserdampf kondensiert und in das Gemisch zurückläuft (Arbeiten mit "totalem Rückfluss"). Die Verdampfung wird durch Druckerniedrigung auf einen Druck unterhalb des Wasserdampfpartialdrucks des Gemischs bei der jeweiligen Temperatur bewirkt. Alternativ kann das Gemisch durch Verdampfen von Wasser aufkonzentriert werden, wobei die Temperatur im Wesentlichen konstant gehalten wird, also der Verdampfungsvorgang im wesentlichen isotherm durchgeführt wird.

Das Gemisch kann durch Verdampfen von Wasser sowohl abgekühlt als auch aufkonzentriert werden. Diese beiden Vorgänge können gleichzeitig oder nacheinander durchgeführt werden. Gleichzeitig finden beide Vorgänge statt, wenn der Verdampfungsvorgang adiabatisch durchgeführt wird und das verdampfte Wasser nicht vollständig ersetzt wird, also z. B. nur mit teilweisem Rückfluss des kondensierten Wassers oder ohne Rückfluss gearbeitet wird. Es kann aber auch zum Beispiel erst eine isotherme Verdampfung unter Konzentrationserhöhung und anschließend eine adiabatische Verdampfung durchgeführt werden, wobei mit totalem, teilweisem Rückfluss oder ohne Rückfluss gearbeitet werden kann.

In einer Ausführungsform des erfindungsgemäßen Verfahrens wird in Schritt (b) durch Verdampfungskühlung abgekühlt, wobei diese solange durchgeführt wird, bis die Temperatur des Gemischs mindestens auf 30 °C abgesunken ist. Weiterhin ist es bevorzugt, Wärme über die Kristallisatorwand abzuführen, sobald eine Temperatur, die bei ca. 30 °C oder darunter liegt, unterschritten wird. Weiterhin bevorzugt ist es, in Schritt (b) eine Abkühlrate, die im zeitlichen Mittel mindestens 5 K/h beträgt, vorzugsweise ≥ 7,5 K/h beträgt, insbesondere im Bereich von 10 bis 30 K/h liegt, einzuhalten.

Bevorzugt wird in Schritt (b) auf eine Temperatur unterhalb von 20 °C abgekühlt. Hierdurch wird eine weitgehende Kristallisation des gelösten MGDN erreicht.

Erfindungsgemäß werden insgesamt größere Kristalle gebildet. An diesen haftet aber weniger Mutterlauge an, insbesondere kann keine Mutterlauge in Agglomeraten feinster Kristalle "eingeschlossen" werden, bzw. die anhaftende Mutterlauge kann leicht abgetrennt werden, z. B. durch einfaches Filtrieren oder Zentrifugieren. Der Reinigungsaufwand verringert sich dadurch erheblich. Auch wird eine Verkrustung der Wände des Kristallisators wirksam vermieden.

Die MGDN-Konzentration des wässrigen Rohproduktgemischs beträgt im Allgemeinen 3 bis 50 Gew.%, bevorzugt 15 bis 40 Gew.%, besonders bevorzugt 20 bis 35 Gew.-%. Ein Gehalt an gelöstem MGDN von 1 Gew.-% entspricht einer Kristallisationstemperatur von ca. 20 °C, ein Gehalt an gelöstem MGDN von 5 Gew.-% einer Kristallisationstemperatur von ca. 55 °C. Bei 10 °C beträgt die Wasserlöslichkeit von MGDN noch etwa 0,5 Gew.-%.

Bei nur geringen MGDN-Gehalten des Gemischs von im Allgemeinen bis zu 10 Gew.-% kann es zu Beginn des Kristallisationsvorgangs zweckmäßig sein, eine so genannte "Impfschleife" durchzuführen, das heißt das Gemisch nach Kristallbildung zunächst wieder um einige °C zu erwärmen, um ein Teil der gebildeten Kristalle wieder aufzulösen, und anschließend erneut abzukühlen.

In einer Ausführungsform des erfindungsgemäßen Verfahren wird in Schritt (b) das wässrige Gemisch durch Verdampfung von Wasser abgekühlt, wobei die MGDN-Konzentration des Gemischs im Wesentlichen konstant gehalten wird. Diese Variante kann auch als "Vakuumkühlungskristallisation" bezeichnet werden, die mit totalem Rücklauf durchgeführt wird.

In einer weiteren Ausführungsform des erfindungsgemäßen Verfahrens wird in Schritt (b) das wässrige Gemisch durch Verdampfen von Wasser aufkonzentriert, wobei die Temperatur des Gemischs im Wesentlichen konstant gehalten wird. Diese Variante kann auch als "isotherme Verdampfungskristallisation" bezeichnet werden.

In einer weiteren Ausführungsform des erfindungsgemäßen Verfahrens umfasst Schritt (b) sowohl Abkühlung als auch Aufkonzentrierung des wässrigen Gemischs, wobei diese beiden Vorgänge gleichzeitig oder nacheinander erfolgen können. In einer bevorzugten Variante dieser Ausführungsform wird eine Vakuumkühlkristallisation mit nur teilweisem Rücklauf des kondensierten Wasserdampfes oder ohne Rücklauf durchgeführt.

Die Ausführung des Kristallisators, in dem das erfindungsgemäße Verfahren durchgeführt wird, ist beliebig. Es kann sich dabei z. B. um einen Rührkessel-, Zwangsumlauf-, Leitrohr- oder Fließbett-Kristallisator, beispielsweise vom Oslo-Typ, handeln. Das erfindungsgemäße Verfahren kann diskontinuierlich, halbkontinuierlich oder kontinuierlich durchgeführt werden. Bevorzugt wird es diskontinuierlich durchgeführt, beispielsweise in einem Rührkessel-Kristallisator.

Die Abtrennung des Kristallisats durch Fest/Flüssig-Trennung kann mit einem beliebigen Fest/Flüssig-Trennapparat, z.B. einer Nutsche, einem Drehfilter, einem Bandfilter, einer Schubzentrifuge, einer Schälzentrifuge oder ähnlichem erfolgen. Das abgetrennte Kristallisat kann anschließend gewaschen werden, beispielsweise mit Wasser oder dem Ausgangsgemisch der Kristallisation als Waschflüssigkeit.

Das MGDN enthaltende wässrige Rohproduktgemisch, aus dem mit dem erfindungsgemäßen Verfahren MGDN isoliert wird, wird im Allgemeinen erhalten durch
1. Umsetzung von Iminodiacetonitril (IDN) mit HCN und Acetaldehyd in wässriger Lösung. Iminodiacetonitril kann in einer vorgelagerten Stufe aus Urotropin und Blausäure oder aus Formaldehydcyanhydrin und Ammoniak als wässrige Emulsion erhalten werden;
2. Umsetzung von Alaninnitril, mit HCN und Formaldehyd in wässriger Lösung. Alaninnitril kann in einer vorgelagerten Stufe aus Acetaldehyd, HCN und Ammoniak oder Acetaldehydcyanhydrin und Ammoniak erhalten werden.

Vorzugsweise wird ein MGDN enthaltendes wässriges Rohproduktgemisch wie folgt erhalten:
1a. Iminodiacetonitril (IDN) wird durch Umsetzung von Urotropin, das in situ aus Ammoniak und Formaldehyd erzeugt werden kann, mit Blausäure bei einem pH-Wert von 5,5 bis 6,3, bevorzugt von 5,7 bis 6,1, besonders bevorzugt von 5,8 bis 6,0 und einer Temperatur im Bereich von 20 bis 90°C, bevorzugt von 25 bis 80°C erhalten. Das Molverhältnis Ammoniak : Formaldehyd : Blausäure beträgt im Allgemeinen 1 : 1,5 : 1,5-1,9, die IDN-Konzentration in der erhaltenen wässrigen Emulsion beträgt im Allgemeinen 15 - 40 Gew.%, bevorzugt 20 - 35 Gew.-%. Anschließend wird der pH-Wert der wässrigen IDN-Emulsion mit Mineralsäure auf 2 - 1,0, bevorzugt 1,8 - 1,5, besonders bevorzugt 1,8 - 1,7 eingestellt. Die angesäuerte IDN-Emulsion wird dann mit Acetaldehyd und Blausäure zum MGDN umgesetzt. Das Molverhältnis IDN : Acetaldehyd : HCN beträgt im Allgemeinen 1 : 1-1,2 : 1 - 1,2, bevorzugt 1 : 1,0 - 1,1 : 1,1 - 1,2, die Temperatur bei der Umsetzung beträgt im Allgemeinen 40 - 90 °C, bevorzugt 50 - 80 °C. Die MGDN-Konzentration der erhaltenen wässrigen Emulsion beträgt im Allgemeinen 20 - 50 Gew.%, bevorzugt 25 - 40 Gew.-%. Vorzugsweise wird die wässrige Emulsion vor Durchführung der Kristallisation mit Wasser auf einen MGDN-Gehalt von 15 - 40 Gew.%, bevorzugt 20 -35 Gew.-% verdünnt.

IDN kann auch durch Umsetzung von Formaldehydcyanhydrin mit Ammoniak hergestellt werden. Alternativ kann von kristallinem IDN als Edukt ausgegangen werden, welches in Wasser emulgiert wird.
2a. Alpha-Alaninnitril (AN) wird durch Umsetzung von überschüssigem Ammoniak mit Acetaldehyd und HCN oder durch Umsetzung von Acetaldehydcyanhydrin mit überschüssigem Ammoniak hergestellt, wobei Ammoniak als wässrige Lösung, gasförmig oder in flüssiger Form eingesetzt werden kann. Die Umsetzung kann unter Druck durchgeführt werden. Der überschüssige Ammoniak wird bevorzugt im Vakuum abdestilliert. Das rohe AN wird mit Formaldehyd und Blausäure zu MGDN umsetzt. Dazu wird der pH-Wert der wässrigen AN-Lösung mit Mineralsäure auf 2 - 1,0, bevorzugt auf 1,8 - 1,5, besonders bevorzugt auf 1,8 - 1,7 einstellt. Das Molverhältnis AN : Formaldehyd : HCN beträgt im Allgemeinen 1 : 1,0 - 1,2 : 1,0 - 1,2, die Temperatur bei der Umsetzung beträgt im Allgemeinen 40 - 90°C, bevorzugt 50 - 80 °C. Die MGDN-Konzentration der erhaltenen wässrigen Emulsion beträgt im Allgemeinen 20 - 50 Gew.-%, bevorzugt 25 - 40 Gew.-%. Vorzugsweise wird die wässrige Emulsion vor Durchführung der Kristallisation mit Wasser auf einen MGDN-Gehalt von 15 - 40 Gew.-%, bevorzugt 20 - 35 Gew.-% verdünnt.

Die Erfindung wird durch die nachfolgenden Beispiele näher erläutert.

### Beispiele

### Herstellung einer wässrigen MGDN-Rohemulsion

### Beispiel 1

IDN wird durch Umsetzung einer Lösung von 173,9 g (1,241 mol) Urotropin in 535 g Wasser mit 210,9 g (7,814 mol) Blausäure hergestellt. Zur Regelung des pH-Wertes auf 5,8 - 5,9 werden insgesamt 188 g 50 gew.-%ige Schwefelsäure zudosiert. Es resultieren 1090 g einer wässrigen Lösung, die 336,3 g (3,54 mol) IDN enthält (95% Ausbeute bezogen auf Formaldehyd). Der pH-Wert der Lösung wird durch Zugabe von 60 g (0,306 mol) 50 gew.-%iger Schwefelsäure auf 1,8 und ihre Temperatur auf 60°C eingestellt. Anschließend werden innerhalb von 75 Minuten 105,1 g (3,894 mol) HCN und 171,6 g (3,894 mol) Acetaldehyd zudosiert. Die Temperatur steigt auf ca. 80°C. Es wird noch 60 Minuten bei 80°C weitergerührt. Der pH-Wert fällt während der Zugabe der Reaktanten und während der Nachreaktion auf ca. 1,2. Es resultieren ca. 1440 g einer ca. 36 gew.-%ige MGDN-Emulsion, welche 515 g MGDN enthält (3,469 mol; 98% Ausbeute bezogen auf IDN).

Isolierung von MGDN aus der Rohemulsion

### Vergleichsbeispiel

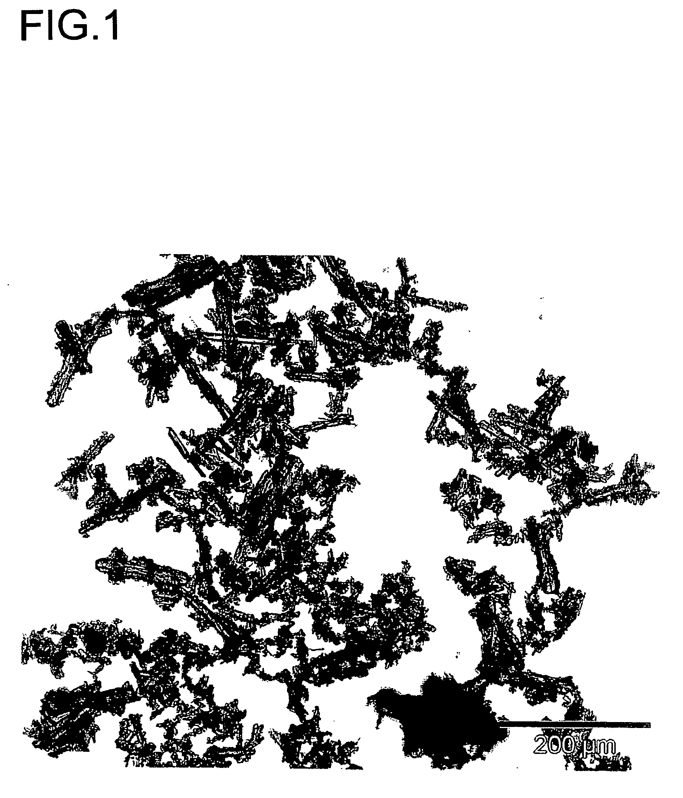

In einem 1-Liter-Doppelmantel-Rührgefäß aus Glas als Kristallisator werden 1120 g einer 8 gew.-%igen, wässrigen MGDN-Emulsion bei 70 °C unter Rühren vorgelegt. Über die Temperierflüssigkeit im Doppelmantel wird die Lösung auf 61 °C abgekühlt. Dabei entstehen sehr viele Kristallkeime. Nach Aufheizen auf 64 °C knapp unterhalb des Erstarrungspunktes verschwindet ein Teil dieser Keime wieder. Anschließend wird unter Rühren mit einer Abkühlrate von 10 K/h durch Solekühlung bis auf 10 °C abgekühlt. Das ausgefallene Kristallisat wird mit einer Nutsche abgetrennt. Wie unter dem Mikroskop (Figur 1) sichtbar wird, enthält das Kristallisat neben ca. 100 µm langen nadelförmigen Kristallen überwiegend Feinanteil einer Größe < 10 µm. Da die Kristalle zur Agglomeration neigen und zwischen den Kristallen Mutterlauge anhaftet, nimmt das feuchte Kristallisat die stark braune Färbung der Mutterlauge an. Die Behälterwände des Kristallisators weisen starke Verkrustungen auf.

### Beispiel 2

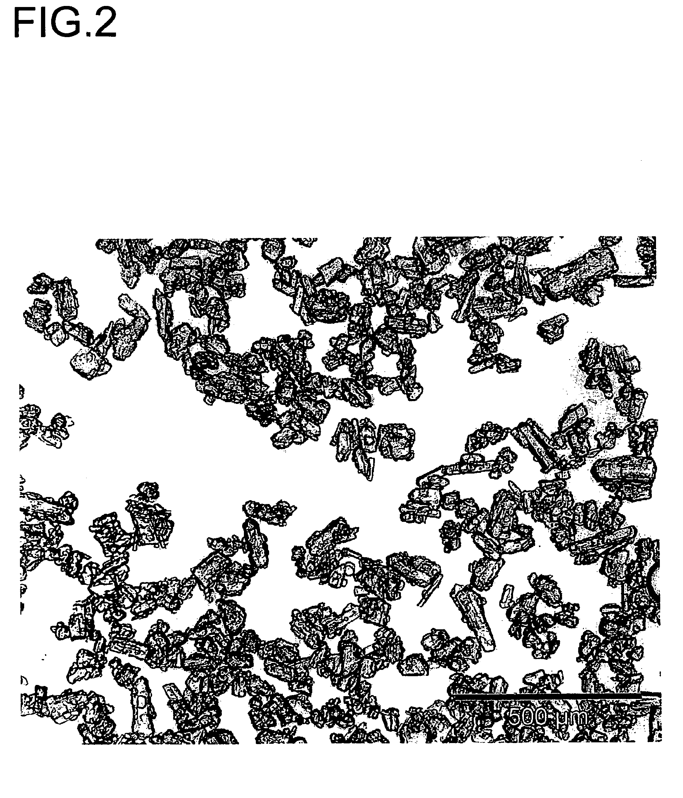

In dem 1-Liter-Doppelmantel-Rührgefäß werden 1970 g einer 22 gew.-%igen wässrigen MGDN-Emulsion bei 70 °C vorgelegt. Über die Temperierflüssigkeit im Doppelmantel wird die Lösung auf 57 °C abgekühlt. Dabei bilden sich Kristallkeime. Durch Aufheizen auf 58 °C wird ein Teil des gebildeten Feststoffs wieder in Lösung gebracht. Anschließend wird Vakuum angelegt und bei 58 °C und 170 mbar zweieinhalb Stunden lang Wasser mit einer Abdampfrate von 250 g/h verdampft. Danach wird der Druck im Kristallisator weiter auf 40 mbar abgesenkt. Der abgezogene Wasserdampf wird nun kondensiert und fließt als Kondensat vollständig in den Kristallisator zurück. Innerhalb von weiteren 2,5 Stunden wird die Suspension mit einer Rate von 20 K/h von 30 °C auf eine Endtemperatur von 10 °C durch Solekühlung der Behälterwand abgekühlt. Die gebildete kalte Suspension wird mit einer Nutsche filtriert. Der feuchte, beige Filterkuchen wird mit der 1,5-fachen Wassermenge gewaschen, wonach er eine leicht gelbliche Farbe annimmt. Die Analyse der Farbzahl einer 1 gew.-%igen Lösung des erhaltenen Kristallisats in Acetonitril ergibt eine Farbzahl nach Hazen von 48. Wie unter dem Mikroskop (Figur 2) erkennbar ist, besteht ein Großteil des Kristallisats aus dicken, nadelförmigen Kristallen von 100 bis 200 µm Länge und 20 bis 40 µm Dicke. Es ist kein Feinanteil mit einer Größe < 1 µm zu erkennen.

### Beispiel 3

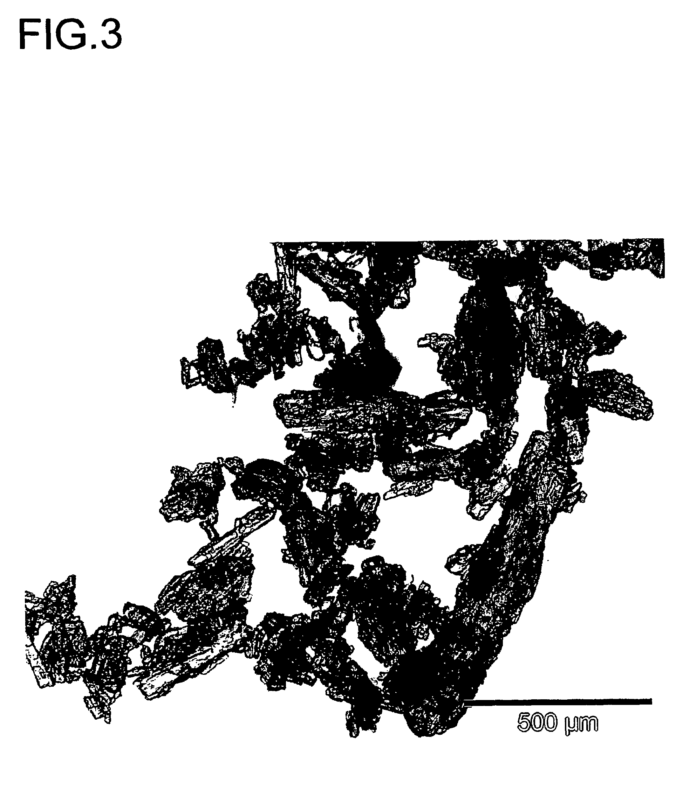

In dem Kristallisator aus Beispiel 1 werden 1300 g einer 30 gew.-%igen wässrigen MGDN-Emulsion bei 70 °C vorgelegt. Nach Unterschreiten des Erstarrungspunktes und einer Haltephase von 40 min bei 60 °C wird durch Verdampfen bei einem Druck von 170 bis 40 mbar und bei totalem Rückfluss des kondensierten Wasserdampfs die Lösung mit einer Rate von 12,5 K/h innerhalb von 4 Stunden auf 10 °C abgekühlt. Ab ca. 30 °C wird dabei die Wärme durch Solekühlung über den Doppelmantel des Kristallisators entzogen. Die Suspension wird in eine Siebbecherzentrifuge gefüllt und bei 2000 min⁻¹ zentrifugiert. Es wird ein hellbeiges Kristallisat erhalten. Unter dem Mikroskop (Figur 3) sind dicke, nadelförmige Kristalle mit eine Länge von bis zu 1 mm und einer Dicke von bis zu 100 µm erkennbar. Feinanteil mit einer Größe < 1 µm ist nicht zu erkennen. Die Analyse der Farbzahl einer 1 gew.-%igen Lösung des Kristallisats in Acetonitril nach Hazen ergibt einen Wert der Farbzahl von 65.

## Patentansprüche

1. Verfahren zur Isolierung von Methylglycinnitril-N,N-diacetonitril (MGDN) aus einer MGDN enthaltenden, wässrigen Emulsion mit einem MGDN-Gehalt von 3 - 50 Gew.-% in einem Kristallisator mit den Schritten:
(a) die wässrige Emulsion wird, ausgehend von einer Temperatur oberhalb des Erstarrungspunktes, auf eine Temperatur unterhalb des Erstarrungspunktes abgekühlt, wobei die Abkühlrate im zeitlichen Mittel 5 K/h nicht übersteigt, bis im Wesentlichen die Gesamtmenge des emulgierten MGDN fest geworden ist,
(b) die erhaltene wässrige Suspension wird weiter abgekühlt und/oder aufkonzentriert, wobei die Abkühlrate größer als in Schritt (a) sein kann.

2. Verfahren nach Anspruch 1, **dadurch gekennzeichnet, dass** in Schritt (a) das wässrige Gemisch durch Verdampfen lassen von Wasser und/oder durch Abführen von Wärme über die Kristallisatorwand abgekühlt wird.

3. Verfahren nach Anspruch 1, **dadurch gekennzeichnet, dass** in Schritt (b) das wässrige Gemisch durch Verdampfen lassen von Wasser abgekühlt und/oder aufkonzentriert wird.

4. Verfahren nach einem der Ansprüche 1 bis 3, **dadurch gekennzeichnet, dass** in Schritt (b) unterhalb einer Temperatur von ≤ 30 °C Wärme über die Kristallisatorwand abgeführt wird.

5. Verfahren nach einem der Ansprüche 1 bis 4, **dadurch gekennzeichnet, dass** in Schritt (b) die Abkühlrate im zeitlichen Mittel mindestens 5 K/h beträgt.

6. Verfahren nach einem der Ansprüche 1 bis 5, **dadurch gekennzeichnet, dass** in Schritt (b) auf eine Temperatur unterhalb 20 °C abgekühlt wird.

7. Verfahren nach einem der Ansprüche 1 bis 6, **dadurch gekennzeichnet, dass** das MGDN enthaltende Rohgemisch erhalten wird durch Umsetzung von Methylglycinnitril mit HCN und Formaldehyd.

## Claims

1. A process for isolating methylglycinenitrile-N,N-diacetonitrile (MGDN) from an aqueous emulsion which comprises MGDN and has an MGDN content of 3-50% by weight in a crystallizer, comprising the steps:
(a) the aqueous emulsion is, starting from a temperature above the solidification point, cooled to a temperature below the solidification point, the cooling rate averaged over time not exceeding 5 K/h, until substantially the entirety of the emulsified MGDN has solidified,
(b) the resulting aqueous suspension is cooled further and/or concentrated, and the cooling rate may be greater than in step (a).

2. The process according to claim 1, wherein the aqueous mixture is cooled in step (a) by allowing water to evaporate and/or by removing heat via the crystallizer wall.

3. The process according to claim 1, wherein the aqueous mixture is cooled and/or concentrated in step (b) by allowing water to evaporate.

4. The process according to any of claims 1 to 3, wherein heat is removed via the crystallizer wall in step (b) below a temperature of ≤ 30°C.

5. The process according to any of claims 1 to 4, wherein the cooling rate averaged over time in step (b) is at least 5 K/h.

6. The process according to any of claims 1 to 5, wherein cooling is effected in step (b) to a temperature below 20°C.

7. The process according to any of claims 1 to 6, wherein the crude mixture comprising MGDN is obtained by reacting methylglycinenitrile with HCN and formaldehyde.

## Revendications

1. Procédé pour l'isolement de méthylglycine-nitrile-N,N-diacétonitrile (MGDN) à partir d'une émulsion aqueuse contenant du MGDN, présentant une teneur en MGDN de 3-50% en poids dans un cristallisateur, présentant les étapes :
(a) l'émulsion aqueuse, partant d'une température supérieure au point de solidification, est refroidie à une température inférieure au point de solidification, la vitesse de refroidissement ne dépassant pas, en moyenne dans le temps, 5 K/h, jusqu'à ce qu'essentiellement la quantité totale du MGDN émulsionné soit devenue solide,
(b) la suspension aqueuse obtenue est refroidie davantage et/ou concentrée, la vitesse de refroidissement pouvant être supérieure à celle de l'étape (a).

2. Procédé selon la revendication 1, **caractérisé en ce que** dans l'étape (a) le mélange aqueux est refroidi en laissant s'évaporer l'eau et/ou par évacuation de chaleur via la paroi du cristallisateur.

3. Procédé selon la revendication 1, **caractérisé en ce que** dans l'étape (b) le mélange aqueux est refroidi et/ou concentré en laissant s'évaporer l'eau.

4. Procédé selon l'une quelconque des revendications 1 à 3, **caractérisé en ce que** dans l'étape (b), sous une température ≤ 30°C, de la chaleur est évacuée via la paroi du cristallisateur.

5. Procédé selon l'une quelconque des revendications 1 à 4, **caractérisé en ce que** dans l'étape (b) la vitesse de refroidissement, en moyenne dans le temps, est d'au moins 5 K/h.

6. Procédé selon l'une quelconque des revendications 1 à 5, **caractérisé en ce que** dans l'étape (b) on refroidit à une température inférieure à 20°C.

7. Procédé selon l'une quelconque des revendications 1 à 6, **caractérisé en ce que** le mélange brut contenant du MGDN est obtenu par transformation de méthylglycine-nitrile avec du HCN et du formaldéhyde.
